(19) **Europäisches Patentamt / European Patent Office / Office européen des brevets**

(11) **EP 2 050 382 A1**

(12) **EUROPEAN PATENT APPLICATION**
published in accordance with Art. 153(4) EPC

(43) Date of publication:
**22.04.2009 Bulletin 2009/17**

(21) Application number: **07791368.9**

(22) Date of filing: **26.07.2007**

(51) Int Cl.:
**A61B 1/00** (2006.01)

(86) International application number:
**PCT/JP2007/064668**

(87) International publication number:
**WO 2008/018300 (14.02.2008 Gazette 2008/07)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **09.08.2006 JP 2006217205**
**30.10.2006 JP 2006293947**

(71) Applicant: **OLYMPUS MEDICAL SYSTEMS CORP.**
**Shibuya-ku**
**Tokyo 151-0072 (JP)**

(72) Inventors:
• **SEGAWA, Hidetake**
**Shibuya-ku, Tokyo 151-0072 (JP)**
• **KOBAYASHI, Satomi**
**Shibuya-ku, Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(54) **CAPSULE ENDOSCOPE**

(57) To provide a capsule endoscope capable of appropriately observing inside a body cavity with reduced oversight, when imaging and observing a large lumen in a subject, a center of gravity is set by a spindle (8) so as to maintain a posture of a capsule body (3) relative to a direction of gravity constant, and an imaging unit (4) is arranged such that an imaging field having an optical axis (φ1) in an oblique direction pointing upward from a horizontal direction is formed with the posture of the capsule body (3) relative to the direction of gravity maintained constant, so that the imaging unit (4) may acquire a central portion of a large intestine (2a), which is a large lumen, by the imaging field having the optical axis (φ1) in the oblique direction pointing slightly upward in a state in which the posture of the capsule body (3) moving while contacting an inner wall of the large intestine (2a) is maintained constant, when imaging and observing inside the large intestine (2a), which is the large lumen in the subject (2), thereby enabling an appropriate observation in a body cavity with reduced oversight.

FIG.1

EP 2 050 382 A1

## Description

TECHNICAL FIELD

The present invention relates to, for example, a capsule endoscope introduced into a lumen organ such as a large intestine in a subject for obtaining an intra-subject image.

BACKGROUND ART

Recently, in a field of the endoscope, the capsule endoscope equipped with an imaging function and a radio communication function has appeared. The capsule endoscope has a configuration to move in organs (in a body cavity) such as an esophagus, a stomach, and a small intestine, with a peristaltic action thereof for observation (examination) during an observation period after being swallowed from a mouth of the subject (human body), until naturally exiting the subject, and to sequentially image using the imaging function.

Patent Document 1: Japanese Patent Application Laid-Open No. 2006-20702

DISCLOSURE OF INVENTION

PROBLEM TO BE SOLVED BY THE INVENTION

However, the conventional capsule endoscope is intended for an observation in the body cavity mainly at a site such as the small intestine, so that when observing a large lumen, such as a large intestine with the capsule endoscope, a center portion of the lumen cannot be acquired with a direct-view-type capsule endoscope due to a large target space, and oversight occurs. Also, when there is a projection, such as a fold of the large intestine, in the lumen, the illumination light is shielded by the fold portion, so that a far side of the lumen is dark and it is hard to observe, and this becomes an obstacle to an appropriate observation in the body cavity.

Also, according to the patent document 1, for preventing an orientation of the capsule endoscope from randomly changing, a weight member such as a power supplying unit for defining a direction of gravity as a reference direction is provided, and an imaging field of an imaging unit is made to include the direction of gravity, so that the imaging field includes a portion that the capsule endoscope contacts, and an inner wall portion of an digestive organ in a pass route can be always imaged; however, this does not solve the above-described problem.

The present invention is achieved in view of the above-description, and an object thereof is to provide the capsule endoscope capable of appropriately observing inside the body cavity with less oversight when imaging and observing the large lumen in the subject.

DISCLOSURE OF INVENTION

PROBLEM TO BE SOLVED BY THE INVENTION

To solve the above problems and achieve the object, a capsule endoscope according to the present invention includes a capsule body to be introduced into a subject; a center-of-gravity setting unit for setting a center of gravity so as to maintain a posture of the capsule body relative to a direction of gravity constant; and an imaging unit arranged in the capsule body such that an imaging field having an optical axis in an oblique direction pointing upward from a horizontal direction is formed with a posture of the capsule body maintained constant relative to the direction of gravity, for imaging a body cavity image.

A capsule endoscope according to the present invention includes a capsule body having a substantially cylindrical shape to be introduced into a subject; a center-of-gravity setting unit for setting a center of gravity such that the capsule body maintains a posture relative to a direction of gravity constant without rotating in a circumferential direction, on a position eccentric from a longitudinal axis of the capsule body; and an imaging unit arranged in the capsule body such that an imaging field having an optical axis in an oblique direction pointing upward from a horizontal direction is formed with a posture of the capsule body maintained constant relative to the direction of gravity, for imaging a body cavity image.

A capsule endoscope according to the present invention includes a capsule body of which specific gravity including a built-in component is set so as to sink in liquid introduced into a subject; a center-of-gravity setting unit for setting a center of gravity so as to maintain a posture of the capsule body relative to a direction of gravity constant in a state in which the capsule body sinks in the liquid introduced into the subject; and an imaging unit arranged in the capsule body such that an imaging field having an optical axis in an oblique direction pointing upward from a horizontal direction is formed with a posture of the capsule body maintained constant relative to the direction of gravity, for imaging a body cavity image.

In the capsule endoscope according to the present invention, the specific gravity of the capsule body including the built-in component is equal to or larger than 1.

In the capsule endoscope according to the present invention, the center-of-gravity setting unit is formed of one member or a combination of a plurality of members built in the capsule body.

In the capsule endoscope according to the present invention, the imaging unit is arranged in the capsule body such that an imaging field is formed in a lumen direction when the capsule body is introduced into a lumen organ of the subject.

In the capsule endoscope according to the present invention, the imaging unit has a diagonal imaging optical system arranged in the capsule body with an optical axis inclined.

In the capsule endoscope according to the present invention, the diagonal imaging optical system is set so as to satisfy a condition, $\alpha < 2\omega/2$ where a diagonal angle of the optical axis is $\alpha$ and a maximum angle of view is $2\omega$.

In the capsule endoscope according to the present invention, the imaging unit has a reflective member for inclining a direction of imaging field forward of an imaging direction of an imaging optical system in which the optical axis is set along the capsule body.

In the capsule endoscope according to the present invention, the imaging optical system is set so as to satisfy a condition, $\alpha < 2\omega/2$ where an inclination angle in a direction of imaging field by the reflective member is $\alpha$ and a maximum angle of view is $2\omega$.

In the capsule endoscope according to the present invention, the imaging unit has imaging fields on both ends in a longitudinal direction of the capsule body.

EFFECT OF THE INVENTION

According to a capsule endoscope according to the present invention, the center of gravity is set so as to maintain the posture of the capsule body relative to the direction of gravity constant, and the imaging unit is arranged so as to form the imaging field having the optical axis in the oblique direction pointing upward from the horizontal direction with the posture of the capsule body maintained constant relative to the direction of gravity, so that when imaging and observing inside the large lumen in the subject, it is possible that the imaging unit acquire the central portion of the large lumen by the imaging field having the optical axis in the oblique direction pointing slightly upward in the state in which the posture of the capsule body, which moves while contacting the inner wall in the lumen, is maintained constant, so that this has the effect of appropriately observing inside the body cavity with the reduced oversight.

BRIEF DESCRIPTION OF DRAWINGS

FIG. 1 is a schematic configuration diagram showing a capsule endoscope according to a first embodiment of the present invention.

FIG. 2-1 is a schematic diagram showing a modification using a battery as a center-of-gravity setting unit.

FIG. 2-2 is a schematic configuration diagram showing a modification using a necessary built-in component as the center-of-gravity setting unit.

FIG. 3 is a schematic configuration diagram showing a binocular type configuration example of the capsule endoscope shown in FIG. 1.

FIG. 4 is a schematic configuration diagram showing the capsule endoscope according to a second embodiment of the present invention.

FIG. 5 is a schematic configuration diagram showing the capsule endoscope according to a third embodiment of the present invention.

FIG. 6 is a schematic configuration diagram showing the capsule endoscope according to a modification.

FIG. 7 is an enlarged schematic configuration diagram showing a vicinity of an end on an imaging side of the capsule endoscope according to a fourth embodiment of the present invention.

FIG. 8 is an illustrative diagram showing a diffusion function of illumination light by a diffuser plate formed of a transmissive member.

FIG. 9 is a characteristic diagram showing a light intensity distribution characteristic on a liquid surface of an LED as a light source of the fourth embodiment and a transmissivity distribution characteristic of a diffuser plate.

FIG. 10-1 is an illustrative diagram showing an example of a configuration of the diffuser plate having a predetermined transmissivity distribution characteristic.

FIG. 10-2 is an illustrative diagram showing another example of the configuration of the diffuser plate having a predetermined transmissivity distribution characteristic.

FIG. 10-3 is an illustrative diagram showing yet another example of the configuration of the diffuser plate having a predetermined transmissivity distribution characteristic.

FIG. 11 is a schematic configuration diagram showing a modification using the diffuser plate formed of a reflective member.

EXPLANATIONS OF LETTERS OR NUMERALS

| 1 | Capsule endoscope |
|---|---|
| 2 | Subject |
| 2a | Large intestine |
| 3 | Capsule body |
| 4 | Imaging unit |
| 4a, 4b | Imaging optical system |
| 6 | Battery |
| 8 | Spindle |
| 9 | Liquid |
| 15a | Prism |
| 21, 31 | Capsule endoscope |

BEST MODE(S) FOR CARRYING OUT THE INVENTION

Hereinafter, embodiments of a capsule endoscope according to the present invention will be described in detail with reference to the drawings. Meanwhile, the present invention is not limited to the embodiments, and various modifications may be made without departing from the sprit of the invention.

First Embodiment

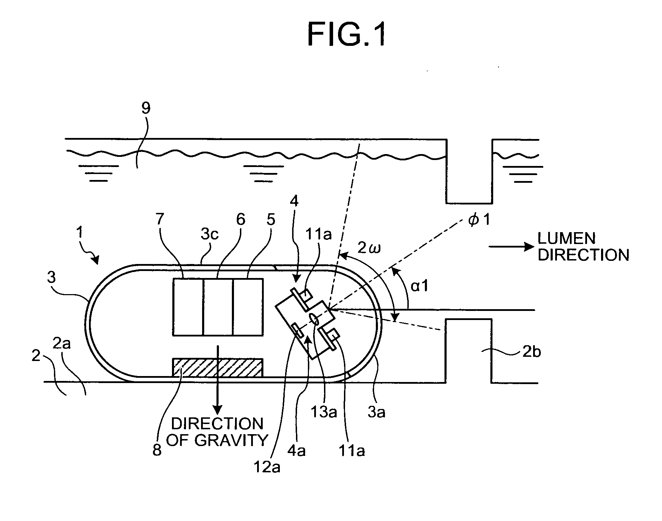

FIG. 1 is a schematic configuration diagram showing the capsule endoscope according to a first embodiment of the present invention. A capsule endoscope 1 of the first embodiment is preferable for imaging and observing inside a body cavity of a subject 2, especially, a large lumen organ such as a large intestine 2a, and is provided with a capsule body 3 insertable into the body cavity of the subject 2, and a built-in components such as an imaging unit 4, a radio transmitter 5, a battery 6, an image processor 7, and a spindle 8, built in the capsule body 3.

The capsule body 3 has a size swallowable from an oral cavity of the subject 2 to the body cavity, and forms an external case in a substantially cylindrical shape for liquid-tightly sealing an interior portion thereof by elastically fitting an end cover 3a in a substantially spherical shape and having transparency or translucency, and a body section cover 3c in a cylindrical shape formed of a colored material through which visible light cannot pass.

Here, the capsule endoscope 1 of the first embodiment moves while sinking in liquid 9 introduced into the subject 2, and specific gravity of the capsule body 3 including the built-in components 4 to 8 thereof is set larger than that of the liquid 9. The liquid 9 is swallowable from the oral cavity of the subject 2, and is transparent relative to a wavelength of a light source used for imaging in the imaging unit 4, and potable water of which specific gravity is near 1 is used, as an example, in the first embodiment. Therefore, the specific gravity of the capsule endoscope 1 is equal to or larger than 1.

The spindle 8 is not a specially required member for a function of the capsule endoscope 1, unlike required members for the function of the capsule endoscope 1 such as the imaging unit 4, the radio transmitter 5, the battery 6, and the image processor 7; however, this is provided for adjusting the specific gravity, and is provided as a center-of-gravity setting unit for setting a center of gravity to a position eccentric from a longitudinal axis so as to maintain a posture of the capsule body 3 sinking in the liquid 9 constant. In the first embodiment, by arranging the spindle 8 on an eccentric position on a lateral axis (direction across a central portion of the longitudinal direction) of the capsule body 3 and on an inner wall surface of the capsule body 3, the center of gravity also is set on the position on the lateral axis and eccentric toward the spindle 8, and the capsule body 3 is set to maintain the posture sinking in the liquid 9 always in a horizontal state without rotating in a circumferential direction in a horizontal portion of the large intestine 2a. Thereby, the posture of the capsule body 3 relative to the direction of gravity is maintained constant such that a side on which the spindle 8 is arranged is a bottom surface portion, and the bottom surface portion is always located on a lower side in the direction of gravity indicated by an arrow, as the posture sinking in the liquid 9.

Also, the imaging unit 4 is imaging means for imaging a body cavity image, and includes an imaging optical system 4a arranged on an end cover 3a side. The imaging optical system 4a is provided with a plurality of light sources 11a each formed of an LED or the like to illuminate an imaging site, an imaging device 12a such as CCD and CMOS imagers for receiving reflected light from the imaging site by illumination light of the light sources 11a to image the body cavity image, and an image forming lens 13a for forming an optical image of the imaging site on the imaging device 12a. A maximum imaging angle of the imaging optical system 4a is set to $2\omega$.

Also, in the first embodiment, the imaging optical system 4a is configured as a diagonal imaging optical system in which an optical axis $\phi1$ is set in an oblique direction pointing upward inclined by a diagonal angle $\alpha1$ from a horizontal direction (longitudinal axis direction of the capsule body 3) in the capsule body 3 of which posture sinking in the liquid 9 is always maintained in the horizontal state and a vertical direction thereof is specified in the horizontal portion of the large intestine 2a. Thereby, an imaging field of the imaging optical system 4a also is set to be in the oblique direction pointing upward, which is a direction opposite to the direction of gravity. As shown in FIG. 1, it is set that the imaging field is generally formed in a lumen direction, as seen from the capsule body 3 on a position having sunk to the inner wall of the large intestine 2a, which is the larger lumen, in the liquid 9.

Here, in the first embodiment, it is set to satisfy a condition, $\alpha1 < 2\omega/2$ where the diagonal angle of the optical axis $\phi1$ of the imaging optical system 4a is $\alpha1$ and the maximum angle of view is $2\omega$. By satisfying this condition, the optical axis $\phi1$ of the imaging optical system 4a does not point too much upward, and an area in a direction downward from the horizontal direction also is included in the imaging field. This allows an observation over an entire lumen including the inner wall bottom surface side portion of the large intestine 2a.

The image processor 7 is for applying a required image process to the body cavity image imaged by the imaging device 12a, and the radio transmitter 5 is for radio outputting data of the body cavity image imaged by the imaging device 12a and to which the required image process is applied by the image processor 7 to a receiver (not shown) arranged outside of the subject 2. The battery 6 is for supplying power required for an electric system driving unit such as the light sources 11a and the imaging device 12a built in the capsule body 3.

Next, the observation of the body cavity image using the capsule endoscope 1 of the first embodiment will be described. Basically, the liquid 9 and the capsule endoscope 1 are swallowed from the oral cavity to fill the large intestine 2a, which is a target site in the subject 2, with the liquid 9, and the body cavity image is imaged by the imaging optical system 4a while allowing the capsule endoscope 1 to move in the filled liquid 9 in the sinking state to observe.

Here, the capsule endoscope 1 is set so as to be eccentric toward the lower portion side in the lateral axis direction by the spindle 8, so that the capsule endoscope 1 is always maintained in the posture horizontal relative to the liquid 9 introduced into the horizontal portion of the target site, which is the large intestine 2a, as shown in FIG. 1, and this does not rotate except about the axis in the direction of gravity. That is to say, the capsule endoscope 1 is maintained in the state in which the bottom surface portion side having the spindle 8 is always located on the lower side in the direction of gravity and the vertical direction thereof is specified, and does not rotate in a circumferential direction. In such constant posture, the imaging field of the imaging optical system 4a is set in the lumen direction according to the optical axis $\phi1$ set in the oblique direction pointing upward from the horizontal direction, so that it is possible to acquire the central portion of the large intestine 2a and to appropriately image and observe inside the large intestine 2a with reduced oversight. At that time, even when there is a projection 2b such as a fold, as shown in FIG. 1, the imaging and observing may be appropriately performed while illuminating a far side thereof with minimum light shielding by the projection 2b.

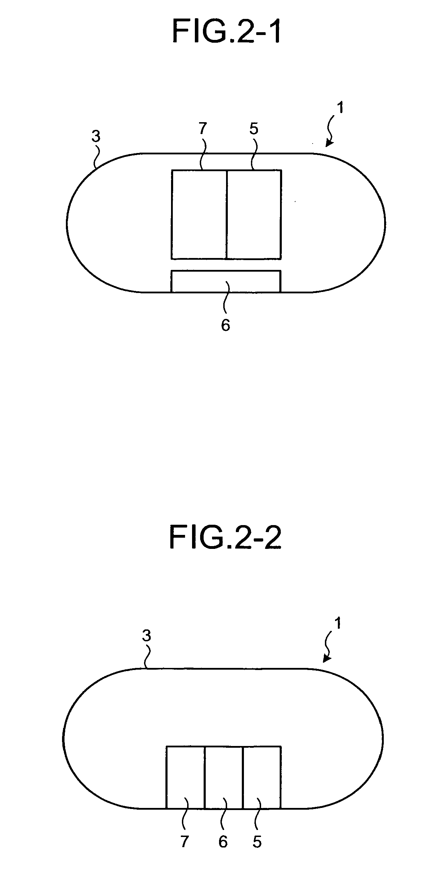

Meanwhile, in the first embodiment, although the center of gravity of the capsule endoscope 1 is set to the eccentric position by additionally providing the spindle 8, which is not necessary for the function of the capsule endoscope 1, as the center-of-gravity setting unit, it is also possible that the battery 6, which is the member necessary for the function of the capsule endoscope 1 and relatively heavy, is used as the center-of-gravity setting unit as shown in FIG. 2-1, for example, in place of the spindle 8, and is arranged on the position on the lateral axis and eccentric from the longitudinal axis of the capsule body 3, thereby eccentrically setting the center of gravity. Alternatively, as shown in FIG. 2-2, the center of gravity may be set on the position on the lateral axis and eccentric from the longitudinal axis of the capsule body 3, by combining the built-in components such as the radio transmitter 5, the battery 6, and the image processor 7, which are a plurality of members necessary for the function of the capsule endoscope 1 and arranging them on one side relative to the longitudinal axis. Such setting of the center of gravity is similar in the following embodiments.

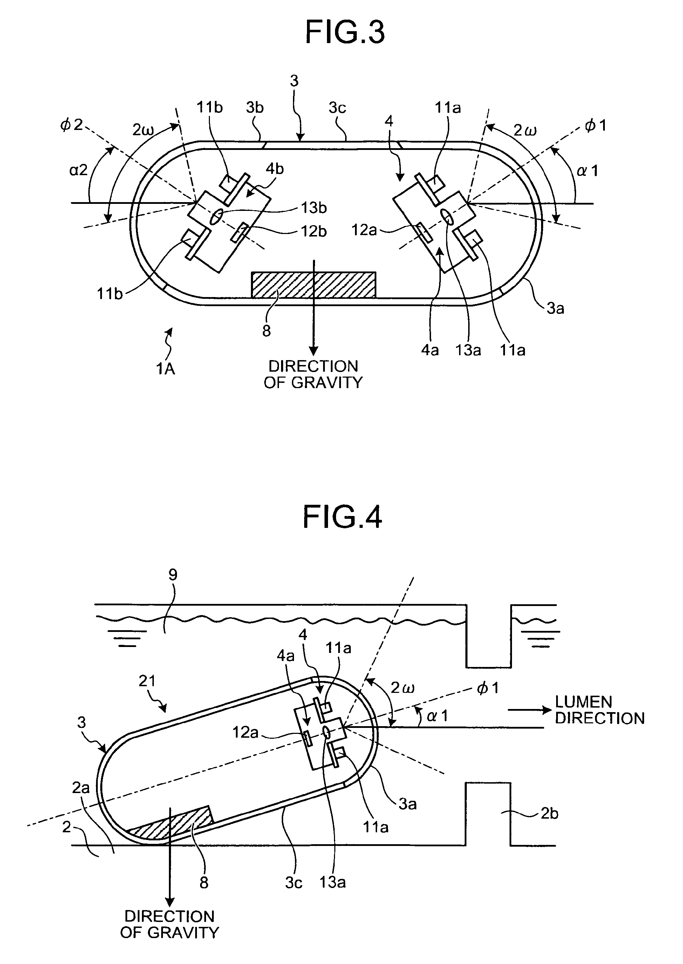

In addition, although it is described as an application example to a monocular capsule endoscope 1 provided with only the diagonal imaging optical system 4a in the first embodiment, it is also possible to configure as a binocular capsule endoscope 1A provided with an imaging optical system 4b similar to the imaging optical system 4a on the other end side in the longitudinal axis direction as shown in FIG. 3, and observe the body cavity image in front-back both directions to reduce the oversight. In FIG. 3, a reference numeral 3b indicates an end cover corresponding to the end cover 3a. Also, the imaging optical system 4b is provided with a plurality of light sources 11b each formed of the LED or the like for illuminating the imaging site, an imaging device 12b such as the CCD and CMOS imagers for receiving the reflected light from the imaging site by the illumination light of the light sources 11b to image the body cavity image, and an image forming lens 13b for forming the optical image of the imaging site on the imaging device 12b. The maximum imaging angle of the imaging optical system 4b is set to $2\omega$.

Here, the imaging optical system 4b is composed as the diagonal imaging optical system in which an optical axis $\phi2$ is set in the oblique direction pointing upward inclined by the diagonal angle $\alpha2$ from the horizontal direction (longitudinal axis direction of the capsule body 3) in the capsule body 3 of which posture sinking in the liquid 9 in the horizontal portion of the large intestine 2a is always maintained in the horizontal state and the vertical direction thereof is specified. Also, this is set so as to satisfy the condition, $\alpha2 < 2\omega/2$ where the diagonal angle of the optical axis $\phi2$ of the imaging optical system 4b is $\alpha2$ and the maximum angle of view is $2\omega$. Meanwhile, the diagonal angles $\alpha1$ and $\alpha2$ of the optical axes $\phi1$ and $\phi2$ of the imaging optical systems 4a and 4b, respectively, may be the same angle or different angles.

Second Embodiment

FIG. 4 is a schematic configuration diagram showing the capsule endoscope according to a second embodiment of the present invention. The portion identical to the portion shown in FIG. 1 is indicated by the same reference numeral. A capsule endoscope 21 of the second embodiment is provided with the capsule body 3 insertable into the body cavity of the subject 2, and the built-in components such as the imaging unit 4 to the spindle 8 built in the capsule body 3. Here, the capsule endoscope 21 of the second embodiment moves in the state sinking in the liquid 9 introduced into the large intestine 2a of the subject 2 as in the case of FIG. 1, and the specific gravity of the capsule body 3 including the built-in components 4 to 8 is set so as to be larger (equal to or larger than 1) as compared to the liquid 9.

In addition, although the imaging unit 4 is configured as the monocular type having the imaging optical system 4a on one side in the longitudinal axis direction of the capsule body 3, in the capsule endoscope 21 of the second embodiment, the optical axis $\phi1$ of the imaging optical system 4a is set in the longitudinal axis direction of the capsule body 3. Further, in the second embodiment, the center of gravity is set by the spindle 8 to the position eccentric from the longitudinal and lateral axes of the capsule body 3 to a backward corner portion (R) side. Thereby, it is set that the posture relative to the direction of gravity is maintained constant in the oblique state such that the portion on which the spindle 8 is arranged becomes the bottom surface portion to contact an inner wall and the end cover 3a side on which the imaging unit 4 is arranged floats from the inner wall in the state sinking in the liquid 9 in the horizontal portion in the large intestine 2a.

The inclination of the capsule body 3 in this case is set such that the optical axis $\phi1$ of the imaging optical system 4a is in the oblique direction pointing upward, which is inclined by the angle $\alpha1$ from the horizontal direction, in the capsule body 3 of which posture sinking in the liquid 9 is always maintained in the inclined state. Thereby, the imaging field of the imaging optical system 4a also is set to the oblique direction pointing upward, which is opposite to the direction of gravity. As shown in FIG. 4, as seen from the capsule body 3 in the position sinking on the inner wall of the large intestine 2a, which is the large lumen, in the liquid 9, it is set that the imaging field is generally formed in the lumen direction.

Here, in the second embodiment, it is set to satisfy the condition, $\alpha1 < 2\omega/2$ where the inclination angle of the optical axis $\phi1$ of the imaging optical system 4a relative to the horizontal direction is $\alpha1$ and the maximum angle of view is $2\omega$. By satisfying this condition, the optical axis $\phi1$ of the imaging optical system 4a does not point too much upward, so that the area in the direction downward from the horizontal direction also is included in the imaging field.

The capsule endoscope 21 according to the second embodiment also has the effect similar to that of the capsule endoscope 1 according to the first embodiment.

Third Embodiment

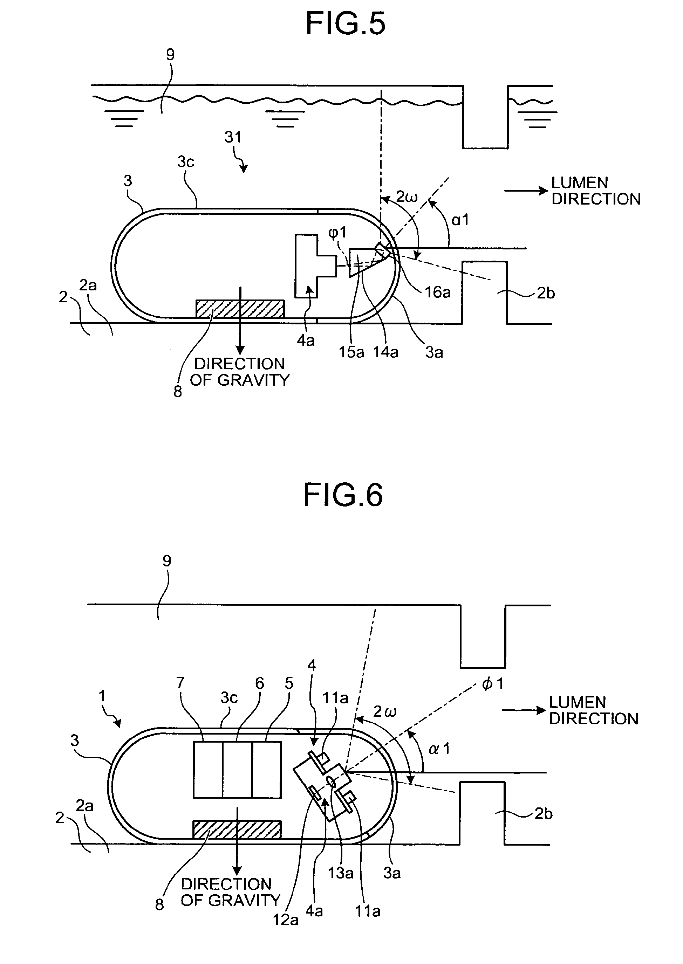

FIG. 5 is a schematic configuration diagram showing the capsule endoscope according to a third embodiment of the present invention. The portion identical to the portion shown in FIG. 1 is indicated by the same reference numeral. A capsule endoscope 31 according to the third embodiment is provided with the capsule body 3 insertable into the body cavity of the subject 2, and the built-in components such as the imaging unit 4 to the spindle 8 built in the capsule body 3. Here, the capsule endoscope 31 of the third embodiment moves in the state sinking in the liquid 9 introduced into the large intestine 2a of the subject 2, as in the case shown in FIG. 1, and the specific gravity of the capsule body 3 including the built-in components 4 to 8 is set so as to be larger (equal to or larger than 1) as compared to that of the liquid 9.

Although the imaging unit 4 is composed as the monocular type having the imaging optical system 4a on one side in the longitudinal axis direction of the capsule body 3, in the capsule endoscope 31 of the third embodiment, the optical axis $\phi1$ of the imaging optical system 4a is set in the longitudinal axis direction of the capsule body 3, and a prism 15a is provided as a reflective member having a reflective surface 14a, which inclines the direction of imaging field of the imaging optical system 4a forward of an imaging direction of the imaging optical system 4a. Here, in the third embodiment, it is set to satisfy the condition, $\alpha1 < 2\omega/2$ where the inclination angle in the direction of imaging field of the imaging optical system 4a by the reflective surface 14a is $\alpha1$ and the maximum angle of view is $2\omega$. By satisfying the condition, the direction of imaging field by the prism 15a does not point too much upward, so that the area in the downward direction from the horizontal direction also is included in the imaging field.

Here, in a case in which the reflective member is the prism 15a, it is required that a refractive index n of a prism glass material satisfies a total reflection condition on a prism refractive surface,

$$n \leq \{1/\sin \ (\pi/2 \ - \ \alpha 1/2 \ - \ \omega)$$

in order to allow a light beam entering from the direction of field of view with the inclination angle $\alpha 1$ to enter the imaging device 12a without loss. It is desirable that the glass material of the prism 15a and the inclination angle of the refractive surface 14a are decided so as to satisfy the total reflection condition. Also, when realizing a wide angle of view, an appropriate glass material may not exist or a prism size may become larger, so that it is desirable to arrange a concave lens 16a on an incident surface of the prism 15a.

The capsule endoscope 31 according to the third embodiment also has the effect similar to that of the capsule endoscope 1 according to the first embodiment. Especially, according to the third embodiment, the optical axis $\phi 1$ of the imaging optical system 4a is set in the longitudinal axis direction of the capsule body 3, so that this can be easily realized only by appropriately setting the direction of the imaging field by adding the prism 15a or the like to the normal monocular capsule endoscope configuration.

Meanwhile, although the example of imaging and observing in the state that the capsule endoscopes 1, 21 and 31 are sunk in the liquid 9 introduced into the large intestine 2a has been described in the first to third embodiments, the large intestine 2a may be observed without using the liquid 9 also. FIG. 6 is a schematic configuration diagram showing a state in which the inside of the large intestine 2a is imaged and observed with the capsule endoscope 1 without using the liquid 9, as an example.

Fourth Embodiment

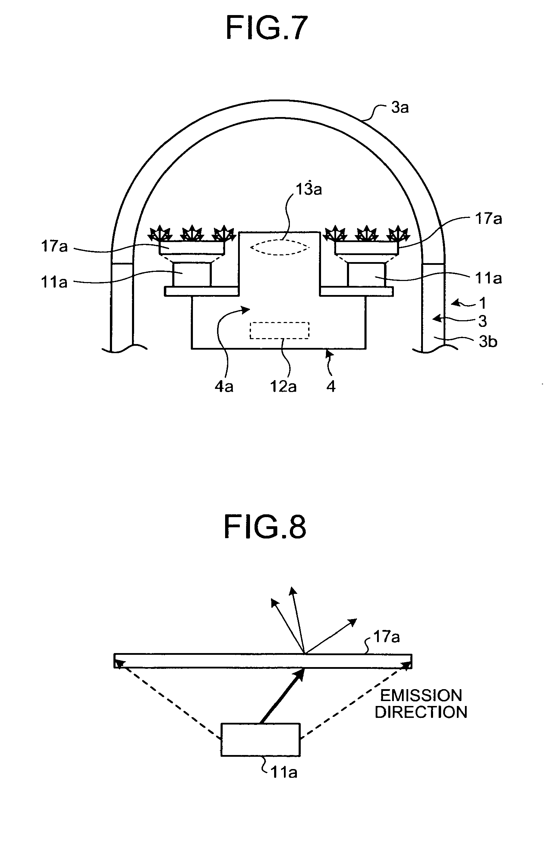

FIG. 7 is an enlarged schematic configuration diagram showing a vicinity of an end portion on an imaging side of the capsule endoscope according to a fourth embodiment of the present invention. The fourth embodiment shows, for example, a configuration example of the imaging optical system 4a according to the first embodiment. The imaging optical system 4a of the fourth embodiment is further provided with a diffuser plate 17a formed of a transmissive member as diffusing means arranged in an emission direction of the illumination light from each of the light sources 11a for diffusing the illumination light from the light sources 11a, as shown in FIG. 7. FIG. 8 is an illustrative view showing a diffusing function of the illumination light by the diffuser plate 17a formed of the transmissive member, and a diffusing agent, for example, of a granular substance may be added for improving diffuseness. Four light sources 11a using the LEDs are arranged around the image forming lens 13a so as to be in a cross shape relative to the center of the optical axis in the imaging unit 4, and the diffusing plates 17a are provided for each of the light sources 11a, and are arranged so as to cover an entire area of an imaging field E.

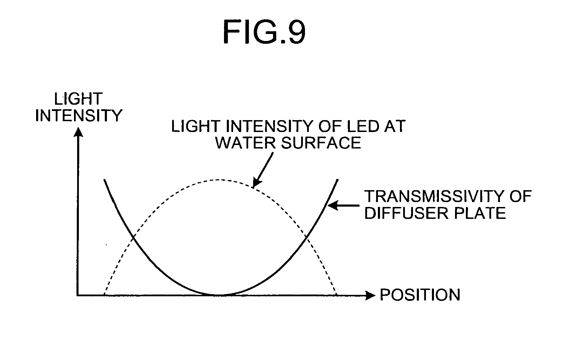

FIG. 9 is a characteristic diagram showing a light intensity distribution characteristic on the liquid 9 surface (water surface) and a transmissivity distribution characteristic of the diffuser plate 17a, when using the LEDs as the light sources 11a of the fourth embodiment. In the fourth embodiment, the diffuser plate 17a having the transmissivity distribution characteristic in which transmissivity increases from the central portion to the peripheral portion so as to be opposite to the light intensity characteristic of the LED (light source 11a) on the liquid 9 surface (water surface) of which light intensity decreases from the central portion to the peripheral portion is used. Such a diffuser plate 17a is arranged so as to be centered on the central portion of the emission direction of the illumination light from the LED.

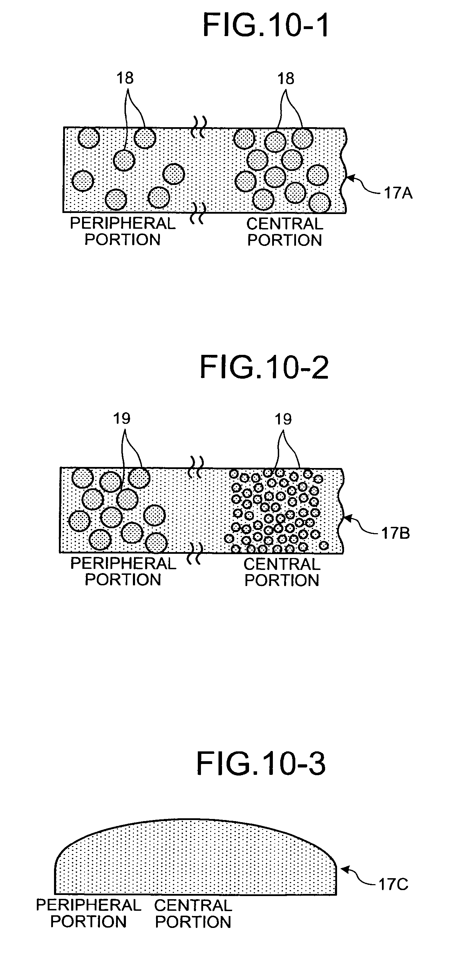

Here, a configuration example of the diffuser plate 17a having the transmissivity distribution characteristic as shown in FIG. 9 is illustrated in FIGS. 10-1 to 10-3. For example, as shown in FIG. 10-1, a diffuser plate 17A in which an additive amount of a diffusing agent 18 increases from the peripheral portion to the central portion may be used. Also, a diffuser plate 17B in which a size of the particle of the diffusing agent 19 to be added becomes smaller from the peripheral portion to the central portion, as shown in FIG. 10-2, may be used. Alternatively, a diffuser plate 17C of which thickness becomes larger from the peripheral portion to the central portion as shown in FIG. 10-3 may be used. In a case of the diffuser plate 17C, since this is not formed of a single crystal, this does not have the lens function even when this has the shape as shown.

In such a configuration, in the fourth embodiment, for example, a predetermined amount of liquid 9 is introduced into an observation target site such as the large intestine in the subject 2 to extend the same, and at the same time, the capsule endoscope 1 is inserted into the subject 2 and is allowed to float in the sinking state in the liquid 9 to extend the observation target site as shown in FIG. 1, and the inner wall or the like of the large intestine, which is the object, is illuminated by the illumination light from the light source 11a and is imaged by the imaging unit 4 to observe.

Here, in a case in which there is not the diffuser plate 17a in the imaging unit 4, the imaging field is directly illuminated by the LED (light source 11a) having the light intensity distribution characteristic in which the light intensity decreases from the central portion to the peripheral portion as shown in FIG. 9; however, in this case, the illumination light is reflected by the liquid surface of the liquid 9 as a boundary surface, and when imaging the object in gas, the illumination light brightly reflects as a light source image, thereby interfering with the observation. Especially, when the illumination light reflects on the boundary surface, the brightest central potion strongly reflects as a bright point, thereby interfering with the imaging of the imaging site.

Therefore, since the fourth embodiment has the diffuser plate 17a for diffusing the illumination light from the LED (light source 11a) in the emission direction of the illumination light from the LED (light source 11a), the illumination light from the LED (light source 11a) is diffused by the diffuser plate 17a to illuminate the imaging field, thereby preventing the light source image from being directly reflected by the boundary surface to reflect into the imaging device 12a when imaging the site in the subject in gas as the object from the liquid 9 across the boundary surface, so that the site of the subject may be appropriately observed. Especially, as in the fourth embodiment, to the normal LED (light source 11a) emitting the illumination light having the light intensity distribution characteristic in which the light intensity decreases from the central portion to the peripheral portion, the diffuser plate 17a formed of the transmissive member having the opposite transmissivity distribution characteristic in which the transmissivity increases from the central portion to the peripheral portion is arranged so as to be centered to the center portion in the emission direction of the illumination light from the LED (light source 11a), the shape of the LED (light source 11a) corresponding to the light intensity distribution characteristic is not formed as an image, so that the imaging field can be uniformly illuminated, and the site of the subject may be appropriately observed.

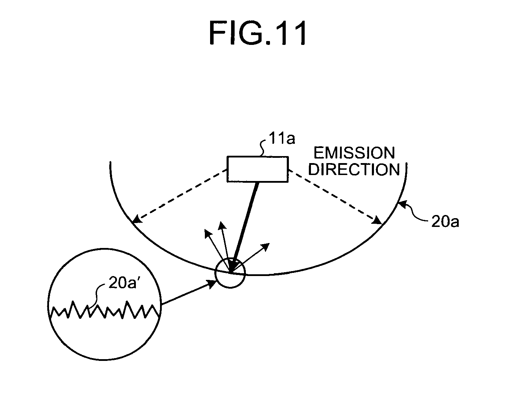

Also, although the diffuser plate 17a formed of the transmissive member through which the illumination light from the LED (light source 11a) passes is used in the fourth embodiment, the diffuser plate formed of the reflective member reflecting the illumination light may be used. FIG. 11 is a schematic configuration diagram showing a modification using a diffuser plate 20a formed of the reflective member. In this case, it is configured that the emission direction of the illumination light of the LED (light source 11a) as the light source is set to be opposite to that in the case in FIG. 2 or the like (pointing downward), and the diffuser plate 20a formed of the reflective member formed in an concave shape with a predetermined curvature is arranged in the emission direction of the illumination light from the LED (light source 11a) to illuminate the imaging field of the imaging unit 4 by reflecting the illumination light upward in a diffusing manner by the diffusing plate 20a. Here, the diffuser plate 20a is configured to reflect the illumination light from the LED (light source 11a) while diffusing the same, by a reflective inner surface thereof formed as a rough surface 20a' as shown in an enlarged view in FIG. 11. In this case, it is desirable that the diffuser plate 20a formed of the reflective member has the reflectivity distribution characteristic in which the reflectivity increases from the central portion to the peripheral portion.

In this manner, by using the diffuser plate 20a of which reflective surface having the predetermined curvature is formed as the rough surface 20a' also, it is possible to diffuse the illumination light from the LED (light source 11a) to uniformly illuminate the imaging field as the light having the intensity distribution substantially the same, and the object in the gas can be observed across the boundary surface without being adversely affected by the reflection on the boundary surface.

INDUSTRIAL APPLICABILITY

As described above, a capsule endoscope according to the present invention is useful in imaging and observing inside the large lumen in the subject, and especially suitable for observing the large intestine.

**Claims**

**1.** A capsule endoscope, comprising:

> a capsule body to be introduced into a subject;
> a center-of-gravity setting unit for setting a center of gravity so as to maintain a posture of the capsule body relative to a direction of gravity constant; and
> an imaging unit arranged in the capsule body such that an imaging field having an optical axis in an oblique direction pointing upward from a horizontal direction is formed with a posture of the capsule body maintained constant relative to the direction of gravity, for imaging a body cavity image.

**2.** A capsule endoscope, comprising:

> a capsule body having a substantially cylindrical shape to be introduced into a subject;
> a center-of-gravity setting unit for setting a center of gravity such that the capsule body maintains a posture relative to a direction of gravity constant without rotating in a circumferential direction, on a position eccentric from a longitudinal axis of the capsule body; and

an imaging unit arranged in the capsule body such that an imaging field having an optical axis in an oblique direction pointing upward from a horizontal direction is formed with a posture of the capsule body maintained constant relative to the direction of gravity, for imaging a body cavity image.

3. A capsule endoscope, comprising:

a capsule body of which specific gravity including a built-in component is set so as to sink in liquid introduced into a subject;

a center-of-gravity setting unit for setting a center of gravity so as to maintain a posture of the capsule body relative to a direction of gravity constant in a state in which the capsule body sinks in the liquid introduced into the subject; and

an imaging unit arranged in the capsule body such that an imaging field having an optical axis in an oblique direction pointing upward from a horizontal direction is formed with a posture of the capsule body maintained constant relative to the direction of gravity, for imaging a body cavity image.

4. The capsule endoscope according to claim 3, wherein the specific gravity of the capsule body including the built-in component is equal to or larger than 1.

5. The capsule endoscope according to any one of claims 1 to 4, wherein the center-of-gravity setting unit is formed of one member or a combination of a plurality of members built in the capsule body.

6. The capsule endoscope according to any one of claims 1 to 5, wherein the imaging unit is arranged in the capsule body such that an imaging field is formed in a lumen direction when the capsule body is introduced into a lumen organ of the subject.

7. The capsule endoscope according to claim 6, wherein the imaging unit has a diagonal imaging optical system arranged in the capsule body with an optical axis inclined.

8. The capsule endoscope according to claim 7, wherein the diagonal imaging optical system is set so as to satisfy a condition, $\alpha < 2\omega/2$ where a diagonal angle of the optical axis is $\alpha$ and a maximum angle of view is $2\omega$.

9. The capsule endoscope according to claim 6, wherein the imaging unit has a reflective member for inclining a direction of imaging field forward of an imaging direction of an imaging optical system in which the optical axis is set along the capsule body.

10. The capsule endoscope according to claim 9, wherein the imaging optical system is set so as to satisfy a condition, $\alpha < 2\omega/2$ where an inclination angle in a direction of imaging field by the reflective member is $\alpha$ and a maximum angle of view is $2\omega$.

11. The capsule endoscope according to any one of claims 1 to 10, wherein the imaging unit has imaging fields on both ends in a longitudinal direction of the capsule body.

# FIG.1

LUMEN
DIRECTION

DIRECTION
OF GRAVITY

# FIG.2-1

# FIG.2-2

# FIG.3

1A

DIRECTION
OF GRAVITY

# FIG.4

LUMEN
DIRECTION

DIRECTION
OF GRAVITY

# FIG.5

# FIG.6

# FIG.7

# FIG.8

EMISSION
DIRECTION

# FIG.9

# FIG.10-1

18                          18

17A

PERIPHERAL
PORTION

CENTRAL
PORTION

# FIG.10-2

19                          19

17B

PERIPHERAL
PORTION

CENTRAL
PORTION

# FIG.10-3

17C

PERIPHERAL    CENTRAL
PORTION       PORTION

# FIG.11

11a

EMISSION DIRECTION

20a

20a'

## INTERNATIONAL SEARCH REPORT

| | International application No. |
|---|---|
| | PCT/JP2007/064668 |

**A. CLASSIFICATION OF SUBJECT MATTER**
*A61B1/00*(2006.01)i

According to International Patent Classification (IPC) or to both national classification and IPC

**B. FIELDS SEARCHED**

Minimum documentation searched (classification system followed by classification symbols)
A61B1/00

Documentation searched other than minimum documentation to the extent that such documents are included in the fields searched
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2003-210394 A  (Olympus Optical Co., Ltd.), | 1-7 |
| Y | 29 July, 2003 (29.07.03), | 8-11 |
| | Par. Nos. [0075], [0079]; Fig. 20 | |
| | (Family: none) | |
| | | |
| A | JP 2005-95602 A  (Given Imaging Ltd.), | 3,4 |
| | 14 April, 2005 (14.04.05), | |
| | Par. No. [0024] | |
| | & WO 2001/010291 A1    & US 2004/0109488 A1 | |
| | & DE 60031501 D        & IL 131242 D | |
| | & AU 6465600 A | |
| | | |
| Y | JP 2003-38425 A  (Olympus Optical Co., Ltd.), | 8,10,11 |
| | 12 February, 2003 (12.02.03), | |
| | Par. No. [0066]; Fig. 14 | |
| | & US 2005/0256372 A1 | |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 October, 2007 (09.10.07) | 23 October, 2007 (23.10.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

## INTERNATIONAL SEARCH REPORT

| | International application No. |
|---|---|
| | PCT/JP2007/064668 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 8-29703 A (Olympus Optical Co., Ltd.), 02 February, 1996 (02.02.96), Par. No. [0052]; Fig. 11 (Family: none) | 9,10 |
| A | JP 2004-357933 A (Olympus Corp.), 24 December, 2004 (24.12.04), Par. Nos. [0016], [0026], [0037]; Figs. 2, 20 & US 2005/0020880 A1 | 1-11 |
| A | JP 2006-149581 A (Olympus Corp.), 15 June, 2006 (15.06.06), Par. Nos. [0038] to [0040]; Fig. 9 & WO 2006/057443 A1 | 1-11 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006020702 A